# EUROPEAN PATENT APPLICATION

(11) **EP 4 715 055 A1**
(43) Date of publication of application: **25.03.2026**
(21) Application number: 24201343.1
(22) Date of filing: 19.09.2024
(51) Int. Cl.: C12N 15/70

(54) **METHOD FOR HIGH EFFICIENCY BACTERIAL IN VIVO OR IN VITRO CLONING**

(71) Applicant: European Molecular Biology Laboratory, 69117 Heidelberg (DE)
(72) Inventor: SAWITZKE, James, 00015 Monterotondo (RM) (IT); CASTILLO CABALLERO, Adriana, 00015 Monterotondo (RM) (IT)
(74) Representative: Krauss, Jan

(57) **Abstract**

The present invention relates to a method for producing a desired linear combined DNA molecule, linear or circular DNA plasmid using at least one genetically altered *E. coli* strain, wherein linear DNA fragments are converted into a linear combined DNA molecule or circular DNA plasmid. The method can be performed *in vivo* or *in vitro,* in particular as a single step method. The invention further relates to a method for cloning a nucleic acid fragment of interest, comprising the method according to the present invention, specific genetically altered *E. coli* strains, a kit for performing the method according to the present invention, and the use of the genetically altered *E. coli* strains, or the kit for producing a linear combined DNA molecule or circular DNA plasmid or cloning a nucleic acid fragment of interest.

## Description

The present invention relates to a method for producing a desired linear combined DNA molecule or circular DNA plasmid using at least one genetically altered *E. coli* strain, wherein linear DNA fragments are converted into a linear combined DNA molecule or circular DNA plasmid. The method can be performed *in vivo* or *in vitro,* in particular as a single step method. The invention further relates to a method for cloning a nucleic acid fragment of interest, comprising the method according to the present invention, specific genetically altered *E. coli* strains, a kit for performing the method according to the present invention, and the use of the genetically altered *E. coli* strains, or the kit for producing a linear combined DNA molecule or circular DNA plasmid or cloning a nucleic acid fragment of interest.

### Background of the invention

Cloning is a fundamental molecular biology technique. With traditional cloning, DNA fragments are created by restriction digests and compatible fragments are ligated together *in vitro.* This mix is then transformed into *E. coli* and the clones must be isolated and screened for correct ones. Thus, with traditional cloning, there are at least two steps and several reagents that must be purchased.

Additionally, how fragments can be joined is limited to the available restriction sites and joined fragments must have compatible ends. Incorrect clones, for example vectors that contain more than one insert occur or multimer plasmids can also be made (see below). Another way to join DNA fragments to create a plasmid clone is NEB Assembly (an improved version of Gibson Assembly). With this method, fragments containing short DNA homologies on their ends are added to a mix of proteins (purchased) that process the fragments, join and ligate them *in vitro.* This mix is next transformed into *E. coli* and the clones must be isolated and checked. Again, incorrect clones can be found from these reactions.

A method for using RecET to directly clone *in vivo* is described in US 2020-0283759A1 (herewith incorporated by reference), and Fu, J., et al. (Full-length RecE enhances linear-linear homologous recombination and facilitates direct cloning for bioprospecting. Nat Biotechnol, 2012. 30(5): p. 440-6), and a recent improvement of this technology was described in Sawitzke, J.A., et al. (Enhancement of RecET-mediated in vivo linear DNA assembly by a xonA mutation. bioRxiv, 2022). The genotype of the *E. coli* strain as used for the method was Δ*lacU169 galK_{TYR145UAG}* Δ*xonA* [λ*cI857 Δ*(*cro-bioA*) (*int-cIII*)<>*gam recE recT*] *IN(rrnD-rrnE)1 rph-1.* Notably, the cells were wild type (WT) for the *recA* gene, allowing rearrangements of plasmids by the endogenous *E. coli* recombination systems, WT for *endA* which produces plasmid DNA that is often degraded during preparation, WT for *hsdR* which allows degradation of unmethlylated DNA.

It is therefore an object of the present invention to provide new strategies for convenient and efficient production of a desired linear combined DNA molecule or plasmids and the cloning of certain desired DNA sequences with high fidelity. Other objects and advantages will become readily apparent to the person of skill when reading the present specification.

The problem of the present invention in a first aspect thereof is solved by a method for producing a linear combined DNA molecule, comprising a) Providing at least one genetically altered *E. coli* strain comprising the genotype Δ*xonA* [λ*cI857* Δ(*cro-bioA*) (*int-cIII*)<>*gam recE recT*], and preferably further comprising *recA1 endA1,* and/or *hsdR* b) suitably transforming the *E. coli* strain of a) with two or more suitable linear DNA fragments to be converted into a linear combined DNA molecule, wherein the linear DNA fragments each comprise a short stretch of overlapping homologous sequences with the other fragment(s), c) suitably culturing the transformed E. *coli* strain of b) and testing and/or selecting for bacteria comprising the linear combined DNA molecule.

Preferred is a method according to the present invention, wherein more than two fragments are combined into one linear combined DNA molecule or a linear plasmid (see, for example, Shintani M, Sanchez ZK, Kimbara K. Genomics of microbial plasmids: classification and identification based on replication and transfer systems and host taxonomy. Front Microbiol. 2015 Mar 31;6:242. doi: 10.3389/fmicb.2015.00242. PMID: 25873913; PMCID: PMC4379921 or Godiska R, Mead D, Dhodda V, Wu C, Hochstein R, Karsi A, Usdin K, Entezam A, Ravin N. Linear plasmid vector for cloning of repetitive or unstable sequences in Escherichia coli. Nucleic Acids Res. 2010 Apr;38(6):e88. doi: 10. 1 093/nar/gkp 1 181. Epub 2009 Dec 29. PMID: 20040575; PMCID: PMC2847241.), or a group of different linear combined DNA fragments is produced.

The problem of the present invention in a second aspect thereof is solved by a method for producing a desired circular DNA plasmid, comprising a) Providing at least one genetically altered *E. coli* strain comprising the genotype Δ*xonA* [λ*cI857* Δ(*cro-bioA*) (*int-cIII*)<>*gam recE recT*], and preferably further comprising *recA1 endA1,* and/or *hsdR,* b) suitably transforming the *E. coli* strain of a) with suitable linear DNA fragments to be converted into a circular DNA plasmid, wherein the linear DNA fragments contain short stretches of homologous sequences to at least one of the other DNA fragments as transformed, c) suitably culturing the transformed *E. coli* strain of b) and testing and/or selecting for bacteria comprising the desired circular DNA plasmid.

Preferred is a method according to the present invention, wherein the DNA fragments as transformed are selected from single-stranded and double-stranded, comprising staggered or blunt ends, and optionally comprising modified nucleic acid bases.

Preferred is a method according to the present invention, wherein the plasmid is a self-replicating plasmid, a shuttle vector, and/or comprises at least one selection marker.

Further preferred is a method according to the present invention, wherein a mix of linear DNA fragments is transformed, comprising four or more, preferably six or more different linear DNA fragments, wherein preferably >10⁴ of desired circular DNA plasmids are produced in one reaction that of these plasmids, >99% are fully correct.

Further preferred is a method according to the present invention, wherein the at least one genetically altered E. *coli* strain is selected from a genotype consisting of
a) F' *proA⁺B⁺ lacI^{q}* Δ*(lacZ)M15 zzf::Tn10 (*Tet^{R}*)*/Δ*(ara-leu) 7697 araD139 fhuA* Δ*lacX74 galK16 galE15 e14- Φ80dlacZΔM15 relA1 nupG rpsL* (Str^{R}) *rph spoT1* Δ*(mrr-hsdRMS-mcrBC)* Δ*xonA* [λ*cI857 Δ(cro-bioA)* (*int-cIII*)<>*gam recE recT*], and preferably further consisting of *recA1* and *endA1*;
b) *Δ(ara-leu)7697 araD139 fhuA ΔlacX74 galK16 galE15 e14- ϕ80dlacZΔM15 relA1 endA1 nupG rpsL (Str^{R}) rph spoT1 Δ(mrr-hsdRMS-mcrBC)* Δ*xonA* [λ*cI857* Δ(*cro-bioA*) (*int-cIII*)<>*gam recE recT*], and preferably further consisting of *recA1* and *endA1*; and
c) *fhuA2Δ(argF-lacZ)U169 phoA glnV44 Φ80Δ(lacZ)M15 gyrA96 relA1 thi-1 hsdR17* Δ*xonA* [λ*cI857* Δ(*cro-bioA*) (*int-cIII*)<>*gam recE recT*], and preferably further consisting of *recA1* and *endA1.*

Another aspect of the invention then relates to a method for cloning a nucleic acid fragment of interest, comprising a method according to the present invention, wherein at least one of the linear DNA fragments to be converted into the DNA molecule or plasmid comprises the nucleic acid fragment of interest.

Another aspect of the invention then relates to a method according to the present invention, further comprising the step of further cultivating the bacteria comprising the desired linear DNA fragment or plasmid, the circular DNA plasmid and/or isolating the desired linear DNA fragment or linear or circular DNA plasmid.

Another aspect of the invention then relates to a genetically altered *E. coli* strain selected from the group consisting of
a) F' *proA⁺B⁺ lacI^{q}* Δ*(lacZ)M15 relA1 zzf::Tn10 (*Tet^{R}*)*/Δ*(ara-leu) 7697 araD139 fhuA* Δ*lacX74 galK16 galE15 e14- Φ80dlacZΔM15 nupG rpsL* (Str^{R}) *rph spoT1* Δ*(mrr-hsdRMS-mcrBC)* Δ*xonA* [λ*cI857* Δ(*cro-bioA*) (*int-cIII*)<>*gam recE recT*]; and preferably further consisting of *recA1* and *endA1*; and
b) *Δ(ara-leu)7697 araD139 fhuA ΔlacX74 galK16 galE15 e14- ϕ80dlacZΔM15 recA1 relA1 endA1 nupG rpsL (Str^{R}) rph spoT1 Δ(mrr-hsdRMS-mcrBC)* Δ*xonA* [λ*cI857* Δ(*cro-bioA*) (*int-cIII*)<>*gam recE recT*], and preferably further consisting of *recA1* and *endA1,*
c) *fhuA2Δ(argF-lacZ)U169 phoA glnV44 Φ80Δ(lacZ)M15 relA1 gyrA96 thi-1 hsdR17* Δ*xonA* [λ*cI857* Δ(*cro-bioA*) (*int-cIII*)<>*gam recE recT*], and preferably further consisting of *recA1* and *endA1*; and
d) a combination thereof.

Another aspect of the invention then relates to a kit comprising at least one genetically altered *E. coli* strain according to the present invention, further comprising at least one of suitable buffers and media, suitable linear DNA fragments for constructing a linear DNA molecule or a circular DNA such as a plasmid for inclusion of a nucleic acid fragment of interest, and optionally user manuals for performing the method according to the present invention.

Another aspect of the invention then relates to a use of a genetically altered *E. coli* strain according to the present invention or the kit according to the present invention for producing a DNA molecule or plasmid or cloning a nucleic acid fragment of interest, according to the present invention.

As mentioned above, the present invention generally relates to a method for producing a desired linear combined DNA molecule or a circular DNA construct, such as a plasmid. The methods first comprise the provision of at least one genetically altered *E. coli* strain comprising the genotype Δ*xonA* [λ*cI857* Δ(*cro-bioA*) (*int-cIII*)<>*gam recE recT*], and preferably further comprising *recA1 endA1.*

The present inventors have now developed a set of engineered *E. coli* bacterial strains and a respective method to provide a new strategy in order to join multiple DNA fragments, for example including a fragment to be cloned.

In the method for producing a linear combined DNA molecule according to the present invention, then, the *E. coli* strain a) is suitably transformed with two or more suitable linear DNA fragments to be converted into a linear combined DNA molecule, wherein the linear DNA fragments each comprise a short stretch of overlapping homologous sequences with the other fragment(s). Each fragment shows homology to the fragment it is joining, and each fragment usually has homology to a different fragment as used. The homologous stretch does not need to be at the end, i.e., can be internal. Two or more linear DNA fragments containing suitable short homologies (not necessarily at their ends), can be directly transformed into these cells in order to assemble the DNAs *in vivo* to generate a linear combined DNA plasmid or molecule.

Preferred is the method according to the present invention, wherein more than two fragments are combined into one linear combined DNA molecule, or a group of different linear combined DNA fragments is produced. As illustrative and simple examples, fragments A, B and C may be used, wherein A overlaps/has homology with B, and B overlaps/has homology with C. As a result, a combined molecule A-B-C is produced. As an alternative, A may be used with B1, B2 and C, wherein A overlaps/has homology with B1 and wherein A overlaps/has homology with B2, B1 and B2 overlap/have homology with C. As a result, a combined molecule A-B1-C and A-B2-C is produced as a mixture. A similar strategy can be applied with circular plasmids.

In the method for producing a desired circular DNA plasmid according to the present invention, then, the *E. coli* strain of a) as above is suitably transformed with suitable linear DNA fragments to be converted into a circular DNA plasmid, wherein the linear DNA fragments contain short stretches of homologous sequences to at least one of the other DNA fragments as transformed.

Each fragment shows homology to the fragment it is joining, and each fragment usually has homology to a different fragment as used. The homologous stretch does not need to be at the end, i.e., can be internal. Two or more linear DNA fragments containing suitable short homologies (not necessarily at their ends), can be directly transformed into these cells in order to assemble the DNAs *in vivo* to generate an intact replicating plasmid.

Finally, the transformed *E. coli* strain of b) as above is suitably cultured and tested and/or selected for bacteria comprising the desired linear combined DNA molecule or plasmid or circular DNA plasmid.

The above technology, herein referred to as "CloneIt" for simplicity, takes place in a single step, and is accurate and efficient. CloneIt is an *in vivo* cloning technique, but also could be performed in vitro, is precise to the base pair, and can be directly compared to similar but more expensive and time-consuming technologies on the market such as Gibson Assembly (Gibson, D.G., et al., Enzymatic assembly of DNA molecules up to several hundred kilobases. Nat Methods, 2009. 6(5): p. 343-5), NEB Assembly, or GeneArt Assembly both in frequency and accuracy.

The method according to the present invention can be performed *in vivo* (within an *E. coli*) or *in vitro* (e.g. in a cell free system) method. Advantageously, the method according to the present invention can be performed as a single step method.

Once the cells are ready for transformation, CloneIt is the fastest, most cost-effective method to create circular DNA plasmids, e.g. from linear double-stranded DNA, including synthetic DNA fragments. In addition, a single-strand oligo can be used as a "fragment" allowing easy cloning of sequences of sgRNA, or shRNA, or pools of them for example.

Preferred is the method according to the present invention, wherein the fragments to be combined into the linear molecule or plasmid have stretches of homologous sequences, i.e. stretches that overlap and are able to form base pairings (hybridize) with the other fragment(s). These stretches need to be of a sufficient length to function in the enzymatic background of the strain that is used, and preferably have a length of between 10 bp to 100 bp, preferably of between 40 bp and 75 bp, and more preferably of about 30 bp to about 50 bp. The homologous sequences (the stretches thereof) can show a base pairing to at least 80%, preferably to at least 90%, more preferably to at least 95%, and most preferably are fully base pairing with at least one of the other DNA fragments as transformed. Herein, base pairing and identity (i.e. a fully complementary strand) are used interchangeably.

The term "homology" or "homologous" refers to sequences showing sequence similarity or identity throughout the region as compared. All methods known to a person skilled in the art can be used to identify sequences that have statistically significant similarity or identity. Percentage identity can be determined by the Blast searches or local alignments; in particular for nucleic acid identity, using, for example, BLAST.

Preferred is the method according to the present invention, wherein the stretches of homologous/identical sequences are located at the end(s) and/or are located inside the DNA fragments as transformed.

Further preferred is the method according to the present invention, wherein the DNA fragments as transformed are selected from single-stranded and double-stranded, comprising staggered or blunt ends.

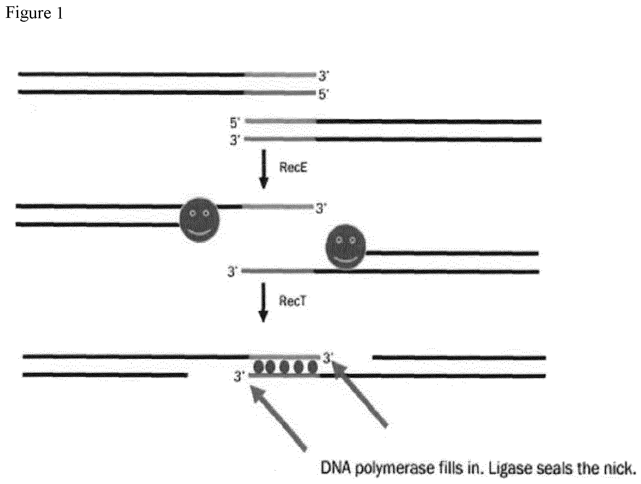

CloneIt is a type of recombineering technology, i.e. genetic engineering using bacteriophage-encoded recombination proteins that are able to act on short homologies (~10-50bp, preferably ~30-50bp). The recombination proteins are expressed by a genetically altered *E. coli* strain. The phage recombination systems typically include two proteins that are expressed and function coordinately (Muyrers, J.P., et al., RecE/RecT and Redα/Redβ initiate double-stranded break repair by specifically interacting with their respective partners (Genes and Development, 2000. 14(15): p. 1971-1982). One protein, a 5'->3' dsDNA-dependent exonuclease (Little, J.W., An exonuclease induced by bacteriophage A. II. Nature of the enzymatic reaction. Journal of Biological Chemistry, 1967. 242(4): p. 679-686), processes intracellular linear dsDNA to leave single-strand 3' overhangs that are bound by the second protein, a single-strand annealing protein (SSAP) (Muniyappa, K. and C.M. Radding, The homologous recombination system of phage λ Pairing activities of β protein. Journal of Biological Chemistry, 1986. 261(16): p. 7472-7478). This SSAP-bound ssDNA anneals to its complementary single-strand sequence; subsequently DNA polymerase I fills in any ssDNA gaps and DNA ligase seals nicks, completing the recombination process, all within the *E. coli* cell (Figure 1). A commonly used recombineering system, RecET, is derived from the *E. coli* cryptic prophage Rac (Gillen, J.R., D.K. Willis, and A.J. Clark, Genetic analysis of the RecE pathway of genetic recombination in Escherichia coli K-12. Journal of Bacteriology, 1981. 145: p. 521-532). RecE protein is the 5'->3' dsDNA-dependent exonuclease (Joseph, J.W. and R. Kolodner, Exonuclease VIII of Escherichia coli. II. Mechanism of action. Journal of Biological Chemistry, 1983. 258(17): p. 10418-10424) and RecT is the SSAP (Hall, S.D., M.F. Kane, and R.D. Kolodner, Identification and characterization of the Escherichia coli RecT protein, a protein encoded by the recE region that promotes renaturation of homologous single-stranded DNA. Journal of Bacteriology, 1993. 175(1): p. 277-287).

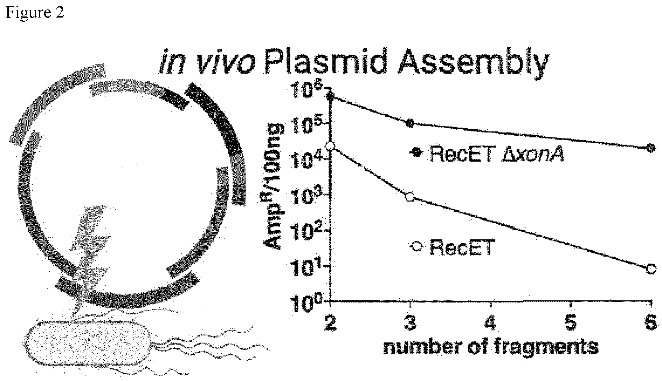

Also, a useful genetic engineering technique is the homology-dependent *in vivo* assembly of multiple linear dsDNAs into a single DNA molecule (Fu, J., et al., Full-length RecE enhances linear-linear homologous recombination and facilitates direct cloning for bioprospecting. Nat Biotechnol, 2012. 30(5): p. 440-6). The linear DNA fragments containing the required homologies (not necessarily on their ends) can be introduced into recombination-proficient cells by transformation (preferably by electroporation) (Thomason, L.C., et al., Recombineering: genetic engineering in bacteria using homologous recombination. Curr Protoc Mol Biol, 2014. 106: p. 1.16.1-39). The RecET recombination system excels at this assembly reaction (Fu, J., et al., Full-length RecE enhances linear-linear homologous recombination and facilitates direct cloning for bioprospecting. Nat Biotechnol, 2012. 30(5): p. 440-6). The inventors have found that deletion of the *E. coli xonA* gene, encoding Exonuclease I, a 3'->5' ssDNA exonuclease (Lehman, I.R. and A.L. Nussbaum, The deoxyribonucleases of Escherichia coli v. on the specificity of exonuclease I (phosphodiesterase). J Biol Chem, 1964. 239: p. 2628-36), further improves the efficiency of this DNA assembly reaction up to 1000-fold (Sawitzke, J.A., et al., Enhancement of RecET-mediated in vivo linear DNA assembly by a xonA mutation. bioRxiv, 2022) (Figure 2).

Particular preferred is the method according to the present invention, wherein a mix of linear DNA fragments is transformed, comprising four or more, preferably six or more different linear DNA fragments, wherein preferably >10⁴ of desired circular DNA plasmids are produced in one reaction that of these plasmids, >99% are fully correct.

Earlier published results (Sawitzke, J.A., et al., Enhancement of RecET-mediated in vivo linear DNA assembly by a xonA mutation. bioRxiv, 2022) are from a strain that has proven to be less than ideal for producing plasmid clones. Although this publication did not analyze the plasmid clones accordingly, the inventors have found that plasmids produced in this strain are most often multimers, i.e. containing two or more copies of the various genes on the plasmid. A common route to multimerization is recombination, but this strain does contain an intact recombination system. However, mutation of the *recA* gene seems not enough to prevent multimer formation when plasmids were engineered by recombineering using the λ Red system (Thomason, L.C., et al., Multicopy plasmid modification with phage λ Red recombineering. Plasmid, 2007. 58(2): p. 148-158).

It was therefore unclear whether a *recA* mutation would fix this significant problem. Multimer plasmids transform less efficiently, reach a lower copy number in cells and are more unstable than monomer plasmids and thus should be avoided. The most important problem regarding multimers is that multimer plasmids made by recombineering may contain a mix of WT and mutant sequences (Thomason, L.C., et al., Multicopy plasmid modification with phage A Red recombineering. Plasmid, 2007. 58(2): p. 148-158).

A plasmid that was constructed as a multimer can be re-engineered into a monomer plasmid by isolating the DNA, cutting it with a restriction enzyme that cuts once, diluting the plasmid before ligation and transformation of the plasmid into a *recA* mutant cell. Then, the correct monomer plasmid can be screened for. This is cumbersome and time-consuming, and it would obviously be better to avoid production of multimers from the start.

This invention has addressed all the above disadvantages by moving the key components, RecET, under regulated control and Δ*xonA* into new genetic backgrounds that are more ideal for cloning. These backgrounds include, for example, *E. coli* NEB5α, NEB Stable, and NEB10β. All strains perform better than the originals, and all new strains make it possible to produce a monomer plasmid, which is extremely important.

In general, any suitable plasmid can be produced using the method according to the present invention. Preferred is the method according to the present invention, wherein the circular or linear plasmid is a self-replicating plasmid, a shuttle vector, and/or comprises at least one selection marker. Additional elements for plasmid construction are provided by the fragments as used, and usually include origin of replication (ori) sequences, and/or mcs, tra sequences or expression cassettes.

In general, any suitable method for transforming DNA sequences (single- or double-stranded) may be used. Preferred is the method according to the present invention, wherein transforming the *E. coli* strain comprises chemical transformation and/or electroporation.

As mentioned above, the method according to the present invention makes use of newly designed genetically altered *E. coli* strains. Preferred is the method according to the present invention, wherein the at least one genetically altered *E. coli* strain is selected from a genotype consisting of
a) F' *proA⁺B⁺ lacI^{q}* Δ*(lacZ)M15 zzf::Tn10 (*Tet^{R}*)*/Δ*(ara-leu) 7697 araD139 fhuA* Δ*lacX74 galK16 galE15 e14- Φ80dlacZΔM15 relA1 nupG rpsL* (Str^{R}) *rph spoT1* Δ*(mrr-hsdRMS-mcrBC)* Δ*xonA* [λ*cI857 Δ(cro-bioA)* (*int-cIII*)<>*gam recE recT*], and preferably further consisting of *recA1* and *endA1*;
b) *Δ(ara-leu)7697 araD139 fhuA ΔlacX74 galK16 galE15 e14- ϕ80dlacZΔM15 relA1 recA1 endA1 nupG rpsL (Str^{R}) rph spoT1 Δ(mrr-hsdRMS-mcrBC)* Δ*xonA* [λ*cI857* Δ(*cro-bioA*) (*int-cIII*)<>*gam recE recT*], and preferably further consisting of *recA1* and *endA1*; and
c) *fhuA2Δ(argF-lacZ)U169 phoA glnV44 Φ80Δ(lacZ)M15 relA1 gyrA96 thi-1 hsdR17* Δ*xonA* [λ*cI857* Δ(*cro-bioA*) (*int-cIII*)<>*gam recE recT*], and preferably further consisting of *recA1* and *endA1.*

It was found in the context of the method according to the present invention that a particularly high activity of the strains could be retained when at least one genetically altered *E. coli* strain was provided as a frozen culture, in particular after 2 or 4 weeks, in particular if the cultures were washed with ice cold water before use thereof (see examples and particularly tables below).

Another aspect of the present invention is a method for cloning a nucleic acid fragment of interest, comprising a method according to the present invention as described herein, wherein at least one of the linear DNA fragments to be converted and/or included into the linear DNA molecule or circular DNA plasmid comprises the nucleic acid fragment of interest. A nucleic acid fragment of interest may comprise an enzyme, a recombinant protein (e.g. antibody) to be produced or the like.

Further preferred is the method according to the present invention, further comprising the step of further cultivating the bacteria comprising the desired DNA molecule or plasmid. This will increase the copy number of the desired DNA molecule or plasmid as produced. Furthermore, the method may further comprise isolating the desired DNA molecule or circular DNA plasmid from the bacterial culture. Methods to isolate linear or circular plasmid DNA are well known to the person of skill, and may include ethanol precipitation and/or gradient centrifugation.

Another aspect of the present invention then relates to a genetically altered *E. coli* strain selected from the group consisting of
a) F' *proA⁺B⁺ lacI^{q}* Δ*(lacZ)M15 zzf::Tn10 (*Tet^{R}*)*/Δ*(ara-leu) 7697 araD139 fhuA* Δ*lacX74 galK16 galE15 e14- Φ80dlacZΔM15 relA1 nupG rpsL* (Str^{R}) *rph spoT1* Δ*(mrr-hsdRMS-mcrBC)* Δ*xonA* [λ*cI857 Δ(cro-bioA)* (*int-cIII*)<>*gam recE recT*]; and preferably further consisting of *recA1* and *endA1*; and
b) *Δ(ara-leu)7697 araD139 fhuA ΔlacX74 galK16 galE15 e14- ϕ80dlacZΔM15 relA1 recA1 endA1 nupG rpsL (Str^{R}) rph spoT1 Δ(mrr-hsdRMS-mcrBC)* Δ*xonA* [λ*cI857* Δ(*cro-bioA*) (*int-cIII*)<>*gam recE recT*], and preferably further consisting of *recA1* and *endA1,*
c) *fhuA2Δ(argF-lacZ)U169 phoA glnV44 Φ80Δ(lacZ)M15 relA1 gyrA96 thi-1 hsdR17* Δ*xonA* [λ*cI857* Δ(*cro-bioA*) (*int-cIII*)<>*gam recE recT*], and preferably further consisting of *recA1* and *endA1*; and
d) a combination thereof.

The inventors have developed a set of genetically modified *E. coli* strains that can be transformed with two or more linear DNA fragments that are then assembled at a high efficiency to generate an intact, precisely engineered linear fragment or plasmid in one step. Correct assembly requires only about 10 or 30bp of sequence homologies that do not have to be on the ends of the recombining fragments. It is precise to the base pair and can be directly compared to similar but more expensive and time-consuming technologies on the market, such as NEB Assembly. Besides standard or complex cloning, this synthetic biology technology can be used for many processes including library creation of mutants for a target of interest for drug discovery, pools of sgRNA for CRISPR screens, and metabolic pathway engineering.

The inventors have found that optimal results can be achieved with as little as 30 ng of each fragment, using homologies of only 10 to 30bp. At least six fragments can be joined to produce >10⁴ plasmids in one reaction, >99% of which are fully correct. (Sawitzke, J.A., et al., Enhancement of RecET-mediated in vivo linear DNA assembly by a xonA mutation. bioRxiv, 2022). This frequency and accuracy is comparable to NEB Assembly.

Another aspect of the present invention then relates to a kit comprising at least one genetically altered *E. coli* strain according to the present invention, further comprising at least one of suitable buffers and media, suitable linear DNA fragments for constructing a linear DNA molecule or plasmid, e.g. for inclusion of a nucleic acid fragment of interest, and optionally user manuals for performing the methods according to the present invention.

Another aspect of the present invention then relates to the use of a genetically altered *E. coli* strain according to the present invention or the kit according to the present invention for producing a DNA molecule or plasmid or cloning a nucleic acid fragment of interest, creating a library of mutants for a target of interest for drug discovery, creating a library of pools of sgRNA for CRISPR screens, and for metabolic pathway engineering.

The present invention relates to the following items:
Item 1. A method for producing a linear combined DNA molecule or plasmid, comprising
   a) Providing at least one genetically altered *E. coli* strain comprising the genotype Δ*xonA* [λ*cI857* Δ(*cro-bioA*) (*int-cIII*)<>*gam recE recT*], and preferably further comprising *recA1 endA1,* and/or *hsdR,*
   b) suitably transforming the *E. coli* strain of a) with two or more suitable linear DNA fragments to be converted into a linear combined DNA molecule or plasmid, wherein the linear DNA fragments each comprise a short stretch of overlapping homologous sequences with the other fragment(s),
   c) suitably culturing the transformed *E. coli* strain of b) and testing and/or selecting for bacteria comprising the linear combined DNA molecule or plasmid.
Item 2. A method for producing a desired circular DNA plasmid, comprising
   a) Providing at least one genetically altered *E. coli* strain comprising the genotype Δ*xonA* [λ*cI857* Δ(*cro-bioA*) (*int-cIII*)<>*gam recE recT*], and preferably further comprising *recA1 endA1,* and/or *hsdR*
   b) suitably transforming the *E. coli* strain of a) with suitable linear DNA fragments to be converted into a circular DNA plasmid, wherein the linear DNA fragments contain short stretches of homologous sequences to at least one of the other DNA fragments as transformed,
   c) suitably culturing the transformed *E. coli* strain of b) and testing and/or selecting for bacteria comprising the desired circular DNA plasmid.
Item 3. The method according to Item 1 or 2, wherein more than two fragments are combined into one linear combined DNA molecule or plasmid, or a group of different linear combined DNA fragments or plasmids is produced.
Item 4. The method according to any one of Items 1 to 3, which is an *in vivo* or *in vitro* method.
Item 5. The method according to any one of Items 1 to 4, which is performed as a single step method.
Item 6. The method according to any one of Items 1 to 5, wherein the stretches of homologous sequences have a length of between 10 bp to 100 bp, preferably of between 40 bp and 75 bp, and more preferably of about 30 bp to about 50 bp, and wherein the homologous sequences show a base pairing to at least 80%, preferably to at least 90%, more preferably to at least 95%, and most preferably are fully pairing with the at least one of the other DNA fragments as transformed.
Item 7. The method according to any one of Items 1 to 6, wherein the stretches of homologous sequences are located at the end(s) and/or are located inside the DNA fragments as transformed.
Item 8. The method according to any one of Items 1 to 7, wherein the DNA fragments as transformed are selected from single-stranded and double-stranded, comprising staggered or blunt ends, and optionally comprising modified nucleic acid bases.
Item 9. The method according to any one of Items 1 to 8, wherein the plasmid is a self-replicating linear or circular plasmid, a shuttle vector, and/or comprises at least one selection marker.
Item 10. The method according to any one of Items 1 to 9, wherein transforming the *E. coli* strain comprises chemical transformation and/or electroporation.
Item 11. The method according to any one of Items 1 to 10, wherein a mix of linear DNA fragments is transformed, comprising four or more, preferably six or more different linear DNA fragments, wherein preferably >10⁴ of desired circular DNA plasmids are produced in one reaction that of these plasmids, >99% are fully correct.
Item 12. The method according to any one of Items 1 to 11, wherein the at least one genetically altered *E. coli* strain is selected from a genotype consisting of
   a) F' *proA⁺B⁺ lacI^{q}* Δ*(lacZ)M15 zzf::Tn10 (*Tet^{R}*)*/Δ*(ara-leu) 7697 araD139 fhuA* Δ*lacX74 galK16 galE15 e14- Φ80dlacZΔM15 relA1 nupG rpsL* (Str^{R}) *rph spoT1* Δ*(mrr-hsdRMS-mcrBC)* Δ*xonA* [λ*cI857 Δ(cro-bioA)* (*int-cIII*)<>*gam recE recT*], and preferably further consisting of *recA1* and *endA1*;
   b) *Δ(ara-leu)7697 araD139 fhuA ΔlacX74 galK16 galE15 e14- ϕ80dlacZΔM15 relA1 recA1 endA1 nupG rpsL (Str^{R}) rph spoT1 Δ(mrr-hsdRMS-mcrBC)* Δ*xonA* [λ*cI857* Δ(*cro-bioA*) (*int-cIII*)<>*gam recE recT*], and preferably further consisting of *recA1* and *endA1*; and
   c) *fhuA2Δ(argF-lacZ)U169 phoA glnV44 Φ80Δ(lacZ)M15 relA1 gyrA96 thi-1 hsdR17* Δ*xonA* [λ*cI857* Δ(*cro-bioA*) (*int-cIII*)<>*gam recE recT*], and preferably further consisting of *recA1* and *endA1.*
Item 13. The method according to any one of Items 1 to 12, wherein the at least one genetically altered *E. coli* strain is provided as a previously frozen culture, in particular after 2 or 4 weeks, and is optionally washed with ice cold water before the use thereof.
Item 14. A method for cloning a nucleic acid fragment of interest, comprising a method according to any one of Items 1 to 13,
   wherein at least one of the linear DNA fragments to be converted into the DNA molecule or plasmid comprises the nucleic acid fragment of interest.
Item 15. The method according to any one of Items 1 to 14, further comprising the step of further cultivating the bacteria comprising the desired DNA molecule or plasmid and/or isolating the desired DNA molecule or plasmid.
Item 16. A genetically altered *E. coli* strain selected from the group consisting of
   a) F' *proA⁺B⁺ lacI^{q}* Δ*(lacZ)M15 zzf::Tn10 (*Tet^{R}*)*/Δ*(ara-leu) 7697 araD139 fhuA* Δ*lacX74 galK16 galE15 e14- Φ80dlacZΔM15 relA1 nupG rpsL* (Str^{R}) *rph spoT1* Δ*(mrr-hsdRMS-mcrBC)* Δ*xonA* [λ*cI857 Δ(cro-bioA)* (*int-cIII*)<>*gam recE recT*]; and preferably further consisting of *recA1* and *endA1*; and
   b) *Δ(ara-leu)7697 araD139 fhuA* Δ*lacX74 galK16 galE15 e14- ϕ80dlacZΔM15 relA1 recA1 endA1 nupG rpsL (Str^{R}) rph spoT1 Δ(mrr-hsdRMS-mcrBC)* Δ*xonA* [λ*cI857* Δ(*cro-bioA*) (*int-cIII*)<>*gam recE recT*], and preferably further consisting of *recA1* and *endA1,*
   c) *fhuA2Δ(argF-lacZ)U169 phoA glnV44 Φ80Δ(lacZ)M15 relA1 gyrA96 thi-1 hsdR17* Δ*xonA* [λ*cI857* Δ(*cro-bioA*) (*int-cIII*)<>*gam recE recT*], and preferably further consisting of *recA1* and *endA1*; and
   d) a combination thereof.
Item 17. A kit comprising at least one genetically altered *E. coli* strain according to Item 16, further comprising at least one of suitable buffers and media, suitable linear DNA fragments for constructing a linear DNA molecule or plasmid for inclusion of a nucleic acid fragment of interest, or user manuals for performing the method according to any one of Items 1 to 15.
Item 18. Use of a genetically altered *E. coli* strain according to Item 16 or the kit according to Item 17 for producing a DNA molecule or plasmid or cloning a nucleic acid fragment of interest, according to any one of claims 1 to 15.

The invention will now be described further in the following examples with reference to the accompanying figures, nevertheless, without being limited thereto. For the purposes of the present invention, all references as cited are incorporated by reference in their entireties.
Figure 1. Schematic mechanism of RecET linear fragment assembly
Figure 2. *In vivo* plasmid assembly. Illustration of how fragments containing 30-50bp of homology are electroporated into cells to construct an intact, replicating plasmid. The "blue" fragments, when assembled, recreate the *lacZ* gene, which can be easily scored for accuracy directly on Petri plates, since plasmids that contain an error-free *lacZ* gene will be blue on indicator plates containing X-gal. On the y-axis are the numbers of plasmids found in cells that produce RecET or ones that produce RecET but are additionally deleted for exonuclease I (Exo I), encoded by the *xonA* gene, as a function of the number of DNA fragments assembled, shown on the x-axis.

### Examples

### Materials and Methods

Similarly to what was disclosed in Sawitzke, J.A., et al. (Enhancement of RecET-mediated in vivo linear DNA assembly by a xonA mutation. bioRxiv), *E. coli* cells were grown with aeration (200rpm) at 32°C until an OD600 of about 0.4 and were further engineered in order to include the *recET* and *gam* recombination genes and additionally mutated for *xonA.*

The *recET* and *gam* genes are in single copy on the bacterial chromosome and under control of the temperature sensitive C*I*857 repressor that controls the strong λ P*_{L}* promoter. Expression of these genes was induced when the cells were quickly switched to 42°C in a shaking water bath (200rpm) for 15 minutes, and then switched off again when the cells were rapidly chilled in an ice water bath. Thus, the recombination genes were expressed for only a short burst. Cells were washed several times with ice old H₂O and concentrated in order to prepare them for electroporation. The cells were then ready for a transformation with linear DNA fragments for use in the method according to the present invention (CloneIt) or were frozen for future use (see below).

### Genetic Engineering of the Clonelt strains according to the present invention

The following *E. coli* strains were generated, the genetic markers that are mentioned during the construction were suitably confirmed.
**JS2270 (NEB Stable version):** F' *proA⁺B⁺ lacI^{q}* Δ*(lacZ)M15 zzf::Tn10 (*Tet^{R}*)*/Δ*(ara-leu) 7697 araD139fhuA ΔlacX74 galK16 galE15 e14- Φ80dlacZ*Δ*M15 relA1 nupG rpsL* (Str^{R}) *rph spoT1 Δ(mrr-hsdRMS-mcrBC)* Δ*xonA* [λ*cI857* Δ(*cro-bioA*) (*int-cIII*)<>*gam recE recT*];
**JS2292 (NEB10β version):** *Δ(ara-leu)7697 araD139 jhuA ΔlacX74 galK16 galE15 e14-ϕ80dlacZΔM15 relA1 nupG rpsL (Str^{R}) rph spoT1 Δ(mrr-hsdRMS-mcrBC)* Δ*xonA* [λ*cI857* Δ(*cro-bioA*) (*int-cIII*)<>*gam recE recT*];
**JS2298 (NEB5α version):** *fhuA2Δ(argF-lacZ)U169 phoA glnV44 Φ80Δ(lacZ)M15 relA1 gyrA96 thi-1 hsdR17* Δ*xonA* [λ*cI857* Δ(*cro-bioA*) (*int-cIII*)<>*gam recE recT*]; and
**Original Clonelt strain:** Δ*lacU169 galK_{TYR145UAG}* Δ*xonA* [λ*cI857* Δ(*cro-bioA*) (*int-cIII*)<>*gam recE recT*] *IN(rrnD-rrnE) 1 rph-1*

### Preparing the Clonelt strains according to the present invention for storage

In the context of the present invention, it was found that the activity after storage of the frozen CloneIt strains according to the present invention can be markedly improved, when following a washing procedure as follows: Cells were thawed on ice, resuspended with 200µl ice cold water, centrifuged, and again resuspended in 50µl (original volume) ice cold water. These cells then used with the standard protocol to join 4 fragments to make a plasmid. JS2298 CloneIt-ready cells were frozen in 5% DMSO. Nevertheless, other cryo-protective treatments as known to the person of skill can be used as well.

| **Cells (JS2298)** | **How treated** | **Clones/10⁸ viable cells** | **Relative activity compared to Fresh cells (%)** |
|---|---|---|---|
| **Freshly made batch #1** | Standard protocol | 8.1×10⁴ | 100 (baseline) |
| | | | |
| **1 week frozen** | Washed 1x* | 8.0×10⁴ | 100 |
| **1 week frozen** | Washed 1x* | 5.4×10⁴ | 66.7 |
| **1 week frozen** | Standard protocol | 1.7×10⁴ | 21 |
| **1 week frozen** | Standard protocol | 1.1×10⁴ | 13.6 |
| | | | |
| **2 weeks frozen** | Washed 1x* | 7.7×10⁴ | 95 |
| **2 weeks frozen** | Washed 1x* | 6.0×10⁴ | 74 |
| **2 weeks frozen** | Standard protocol | 4.7×10³ | 5.8 |
| **2 weeks frozen** | Standard protocol | 7.4×10³ | 9.1 |
| | | | |
| **4 weeks frozen** | Washed 1x* | 1.5×10⁴ | 18.5 |
| **4 weeks frozen** | Washed 1x* | 3.8×10⁴ | 46.9 |
| **4 weeks frozen** | Standard protocol | 3.4×10³ | 4.2 |
| **4 weeks frozen** | Standard protocol | 3.8×10³ | 4.7 |
| | | | |
| **7 weeks frozen** | Washed 1x* | 3.6×10⁴ | 44.4 |
| **7 weeks frozen** | Washed 1x* | 5.6×10⁴ | 69.1 |
| **7 weeks frozen** | Standard protocol | 1.1×10⁴ | 13.6 |
| **7 weeks frozen** | Standard protocol | 2.9×10³ | 3.6 |
| | | | |

| **Freshly made batch #2** | Standard protocol | 6.5×10⁴ | 100 |
|---|---|---|---|
| | | | |
| **1 week frozen** | Washed 1x* | 2.3×10⁴ | 35.4 |
| **1 week frozen** | Standard protocol | 2.6×10³ | 4 |

JS2292 CloneIt-ready cells frozen in 5% DMSO.

| **Cells (JS2292)** | **How treated** | **Clones/10⁸ viable cells** | **Relative activity compared to Fresh cells (%)** |
|---|---|---|---|
| **Freshly made batch #1** | Standard protocol | 4.7×10⁵ | 100 |
| **1 Week frozen** | Washed 1x* | 6.1×10⁵ | 130 |
| **1 week frozen** | Washed 1x* | 6.6×10⁵ | 140 |
| **1 week frozen** | Standard protocol | 2.2×10⁵ | 46.8 |
| **1 week frozen** | Standard protocol | 2.0×10⁵ | 42.6 |
| | | | |

| **Freshly made batch #2** | Standard protocol | 7.3×10⁵ | 100 |
|---|---|---|---|
| **1 week frozen** | Washed 1x* | 6.7×10⁵ | 91.8 |
| **1 week frozen** | Washed 1x* | 6.1×10⁵ | 83.6 |
| **1 week frozen** | Standard protocol | 3.2×10⁵ | 43.8 |
| **1 week frozen** | Standard protocol | 8.8×10⁴ | 12.1 |

| | | | |
|---|---|---|---|
| * Washing procedure: Cells were thawed on ice, resuspended with 200µl ice cold water, centrifuged, and again resuspended in 50µl (original volume) ice cold water. These cells then used with the standard protocol to join 4 fragments to make a plasmid. | | | |

JS2292 exhibited about 10-fold more activity than JS2298, and this higher activity is maintained after freezing. JS2292 maintains sufficient activity after 6 weeks, even without washing. A JS2298 frozen batch has been tested out to 12 weeks, and also maintained sufficient activity.

### Experimental procedures

### Step 1: Linking a drug-resistance marker to the RecET system

The starting *E. coli* strain was transformed with the plasmid pSIM29. This plasmid produces the λ Red recombination proteins when induced, allowing for high frequency recombineering. Although the RecET system as already present in the cells will promote recombineering, it was found that the Red system was superior, and the RecET system was only used in the later steps. Using recombineering in this plasmid-containing strain, a *cat-sacB* cassette was inserted between two bases in the *ybhC* gene which is located right next to the λ *att* site, location of the *recETgam* construct in the chromosome of the starting *E. coli* strain.

This introduced a selectable marker very tightly genetically linked to the RecET system and allowed us to detect the introduction of the RecET system into different strains, as was done in the next step. The strain as produced was designated JS1906.

### Step 2: P1 transduction in order to introduce the RecET system into recipient strains

P1 transduction was used in order to introduce the RecET system into different strains (Thomason, L.C., N. Costantino, and D.L. Court, E. coli genome manipulation by P1 transduction. Curr Protoc Mol Biol, 2007. Chapter 1: p. Unit 117). When the bacteriophage P1 (bacterial virus) is grown on a "donor" strain (in this case JS1906), it makes mistakes, and sometimes pieces of the bacterial chromosome instead of virus DNA are packaged into its capsids. Thus, a lysate will contain viruses that statistically contain all parts of the bacterial chromosome, at ~100kb in each virus. These ~100kb bacterial fragments can be transferred by the P1 bacteriophage into a "recipient" bacterial strain (a process called P1 transduction). As a result, a phage lysate grown on, for example, JS1906 will contain some viruses that contain RecET together with the tightly linked *cat-sacB* cassette.

A lysate of JS1906 was used to transduce the recipient strains, and chloramphenicol resistance as encoded by the *cat-sacB* cassette was selected for. Incorporation of the transduced DNA into the recipient strain requires homologous DNA recombination catalyzed by the *E. coli* recombination system, and RecA as a key component of this recombination system was mutated in all of the recipient strains. Therefore, all recipient strains used herein, i.e., *E. coli* NEB Stable, NEB 10β, and NEB5α were first transformed with pRecA^{ts}, a temperature-sensitive plasmid that produces the WT RecA protein before being transduced with the JS1906 P1 lysate(s).

### Step 3: Confirming that the RecET system was introduced, and linked nearby markers were unchanged

As mentioned above, the *ybhC<>cat-sacB* cassette is tightly genetically linked to the RecET system, and once a strain shows to have acquired the chloramphenicol resistance maker, there is nearly a 100% chance that the strain has acquired the RecET construct as well. The presence of the *recET* genes as part of the expected λ *att* construct were confirmed by PCR using oligos that flank or are within the construct, and the presence of *recE* and *recT* were confirmed in the strains. Full functionality was tested by respective tests as further described herein.

Nevertheless, since phage P1 may transfer ~ 100kb pieces of DNA, any genetic marker that may be within 100kb of RecET potentially could have been moved from the donor strain to the recipient strain as well. In the present case, this could include the *galK* and *galE* markers. Since any *galKam* mutation present in the donor would be undesirable in the recipients, several transduced candidates were screened for their *galK* and *galE* markers in JS2270 and JS2292. To screen the alleles present, a PCR product was made of the *galK* gene. *galK16* is an insertion mutation, so the PCR product was longer than expected for WT or *galKam.* All PCR products were then sequenced, and the presence of *galK* markers was confirmed. The same was done for *galE,* and the confirmed markers are listed in the genotypes above.

### Step 4: Removing cat-sacB next to the RecET construct

The *cat-sacB* cassette is a selectable/counter-selectable marker (Sawitzke, J.A., et al., Recombineering: in vivo genetic engineering in E. coli, S. enterica, and beyond. Methods in Enzymology, 2007. 421: p. 171-199). The inventors used chloramphenicol-resistance in order to select for the cassette in step 2, and now sucrose resistance was selected for in order to remove the cassette (selection against the presence of *sacB*). After the RecET system was confirmed in the new strains, recombineering promoted by RecET and a respective nucleic acid oligo was used in order to precisely remove the *cat-sacB* cassette, restoring this DNA to the WT configuration. Sequencing of the region confirmed that the WT sequence was restored and that no parts of the *cat-sacB* sequence remained. This prepared the strains for the use of the *cat-sacB* cassette again in the next step.

### Steps 5 and 6: Deletion of the xonA gene

In the next step, the *xonA* gene had to be deleted, and the exact mutation as found in the original strain had to be included. This required a two-step process, again using the *cat-sacB* cassette. The *cat-sacB* cassette was first inserted into the *xonA* gene using chloramphenicol-resistance as a selectable marker. The insertion site was then verified by PCR on both ends of the insertion site. In the second step, using a suitable oligonucleotide, RecET mediated recombineering was used to remove the *cat-sacB* cassette, creating the desired deletion. This deletion leaves the first 15 amino acids of *xonA* intact, followed by a stop codon (the full protein would be 475aa). This construct also left the very closely positioned *tsuB* gene intact, and thus fully WT. Again, DNA sequencing confirmed that the desired mutation was introduced.

### Step 7: Removal of the pRecA^{ts} plasmid

For Step 2, a functional *recA* gene was needed, and therefore the pRecA^{ts} plasmid was introduced into the recipient cells that were used in Step 2. Subsequently, it needed to be removed. This was done by growing the cells at 37°C for 4 hours (stopping replication of the pRecA^{ts} plasmid, but also inducing the RecET system), diluting and plating on plates to grow at 30°C (shutting off the RecET system). Individual colonies were checked in order to confirm that they were now ampicillin-sensitive, and thus missing the plasmid.

### Step 8: Confirmation that the new strains are functional for fragment joining

Finally, an assay was designed in order to confirm that the strains as constructed were able to perform fragment joining. The strains were then tested accordingly.

## Claims

1. A method for producing a linear combined DNA molecule or plasmid, comprising
a) Providing at least one genetically altered *E. coli* strain comprising the genotype Δ*xonA* [λ*cI857* Δ(*cro-bioA*) (*int-cIII*)<>*gam recE recT*], and preferably further comprising *recA1 endA1,* and/or *hsdR,*
b) suitably transforming the *E. coli* strain of a) with two or more suitable linear DNA fragments to be converted into a linear combined DNA molecule or plasmid, wherein the linear DNA fragments each comprise a short stretch of overlapping homologous sequences with the other fragment(s),
c) suitably culturing the transformed *E. coli* strain of b) and testing and/or selecting for bacteria comprising the linear combined DNA molecule or plasmid, wherein preferably more than two fragments are combined into one linear combined DNA molecule or plasmid, or a group of different linear combined DNA fragments or plasmids is produced.

2. A method for producing a desired circular DNA plasmid, comprising
a) Providing at least one genetically altered *E. coli* strain comprising the genotype Δ*xonA* [λ*cI857* Δ(*cro-bioA*) (*int-cIII*)<>*gam recE recT*], and preferably further comprising *recA1 endA1,* and/or *hsdR,*
b) suitably transforming the *E. coli* strain of a) with suitable linear DNA fragments to be converted into a circular DNA plasmid, wherein the linear DNA fragments contain short stretches of homologous sequences to at least one of the other DNA fragments as transformed,
c) suitably culturing the transformed *E. coli* strain of b) and testing and/or selecting for bacteria comprising the desired circular DNA plasmid, wherein preferably more than two fragments are combined into one linear combined DNA molecule, or a group of different linear combined DNA fragments is produced.

3. The method according to claim 1 or 2, which is an *in vivo* or *in vitro* method.

4. The method according to any one of claims 1 to 3, wherein the stretches of homologous sequences have a length of between 10 bp to 100 bp, preferably of between 40 bp and 75 bp, and more preferably of about 30 bp to about 50 bp, and wherein the homologous sequences show a base pairing to at least 80%, preferably to at least 90%, more preferably to at least 95%, and most preferably are fully pairing with the at least one of the other DNA fragments as transformed.

5. The method according to any one of claims 1 to 4, wherein the stretches of homologous sequences are located at the end(s) and/or are located inside the DNA fragments as transformed.

6. The method according to any one of claims 1 to 5, wherein the DNA fragments as transformed are selected from single-stranded and double-stranded, comprising staggered or blunt ends, and optionally comprising modified nucleic acid bases.

7. The method according to any one of claims 1 to 6, wherein the plasmid is a self-replicating plasmid, a shuttle vector, and/or comprises at least one selection marker, and/or wherein transforming the E. *coli* strain comprises chemical transformation and/or electroporation.

8. The method according to any one of claims 1 to 7, wherein a mix of linear DNA fragments is transformed, comprising four or more, preferably six or more different linear DNA fragments, wherein preferably >10⁴ of desired circular DNA plasmids are produced in one reaction that are >99% correct.

9. The method according to any one of claims 1 to 8, wherein the at least one genetically altered *E. coli* strain is selected from a genotype consisting of
a) F' *proA⁺B⁺ lacI^{q}* Δ*(lacZ)M15 zzf::Tn10 (*Tet^{R}*)*/Δ*(ara-leu) 7697 araD139 fhuA* Δ*lacX74 galK16 galE15 e14- Φ80dlacZΔM15 relA1 nupG rpsL* (Str^{R}) *rph spoT1* Δ*(mrr-hsdRMS-mcrBC)* Δ*xonA* [λ*cI857 Δ(cro-bioA)* (*int-cIII*)<>*gam recE recT*], and preferably further consisting of *recA1* and *endA1*;
b) *Δ(ara-leu)7697 araD139 fhuA ΔlacX74 galK16 galE15 e14- ϕ80dlacZΔM15 relA1 recA1 endA1 nupG rpsL (Str^{R}) rph spoT1 Δ(mrr-hsdRMS-mcrBC)* Δ*xonA* [λ*cI857* Δ(*cro-bioA*) (*int-cIII*)<>*gam recE recT*], and preferably further consisting of *recA1* and *endA1*; and
c) *fhuA2Δ(argF-lacZ)U169 phoA glnV44 Φ80Δ(lacZ)M15 relA1 gyrA96 thi-1 hsdR17* Δ*xonA* [λ*cI857* Δ(*cro-bioA*) (*int-cIII*)<>*gam recE recT*], and preferably further consisting of *recA1* and *endA1.*

10. The method according to any one of claims 1 to 9, wherein the at least one genetically altered *E. coli* strain is provided as a previously frozen culture, in particular after 2 or 4 weeks, and is optionally washed with ice cold water before the use thereof.

11. A method for cloning a nucleic acid fragment of interest, comprising a method according to any one of claims 1 to 10, wherein at least one of the linear DNA fragments to be converted into the DNA molecule or plasmid comprises the nucleic acid fragment of interest.

12. The method according to any one of claims 1 to 11, further comprising the step of further cultivating the bacteria comprising the desired DNA molecule or plasmid and/or isolating the desired DNA molecule or plasmid.

13. A genetically altered *E. coli* strain selected from the group consisting of
a) F' *proA⁺B⁺ lacI^{q}* Δ*(lacZ)M15 zzf::Tn10 (*Tet^{R}*)*/Δ*(ara-leu) 7697 araD139 fhuA* Δ*lacX74 galK16 galE15 e14- Φ80dlacZΔM15 relA1 nupG rpsL* (Str^{R}) *rph spoT1* Δ*(mrr-hsdRMS-mcrBC)* Δ*xonA* [λ*cI857 Δ(cro-bioA)* (*int-cIII*)<>*gam recE recT*]; and preferably further consisting of *recA1* and *endA1*; and
b) *Δ(ara-leu)7697 araD139 fhuA ΔlacX74 galK16 galE15 e14- ϕ80dlacZΔM15 relA1 recA1 endA1 nupG rpsL (Str^{R}) rph spoT1 Δ(mrr-hsdRMS-mcrBC)* Δ*xonA* [λ*cI857* Δ(*cro-bioA*) (*int-cIII*)<>*gam recE recT*], and preferably further consisting of *recA1* and *endA1,*
c) *fhuA2Δ(argF-lacZ)U169 phoA glnV44 Φ80Δ(lacZ)M15 relA1 gyrA96 thi-1 hsdR17* Δ*xonA* [λ*cI857* Δ(*cro-bioA*) (*int-cIII*)<>*gam recE recT*], and preferably further consisting of *recA1* and *endA1*; and
d) a combination thereof.

14. A kit comprising at least one genetically altered *E. coli* strain according to claim 13, further comprising at least one of suitable buffers and media, suitable linear DNA fragments for constructing a linear DNA molecule or plasmid for inclusion of a nucleic acid fragment of interest, and optionally user manuals for performing the method according to any one of claims 1 to 12.

15. Use of a genetically altered *E. coli* strain according to claim 13 or the kit according to claim 14 for producing a DNA molecule or plasmid or cloning a nucleic acid fragment of interest, according to any one of claims 1 to 13.
